# EUROPÄISCHE PATENTSCHRIFT

(11) **EP 0 901 626 B1**
(45) Veröffentlichungstag und Bekanntmachung des Hinweises auf die Patenterteilung: **17.11.1999**
(21) Anmeldenummer: 97925871.2
(22) Anmeldetag: 23.05.1997
(51) Int. Cl.: G01N 33/38

(54) **VERFAHREN UND VORRICHTUNG ZUR MESSUNG DER FEUCHTE IN BAUSTOFFEN**
METHOD AND DEVICE FOR MEASURING MOISTURE IN BUILDING MATERIALS
PROCEDE ET DISPOSITIF POUR LA MESURE DE L'HUMIDITE DANS LES MATERIAUX DE CONSTRUCTION

(30) Priorität: 24.05.1996 DE 19620912
(43) Veröffentlichungstag der Anmeldung: 17.03.1999
(73) Patentinhaber: Pleyers, Gerd, Dipl.-Ing., 52146 Würselen (DE)
(72) Erfinder: Pleyers, Gerd, Dipl.-Ing., 52146 Würselen (DE)
(74) Vertreter: Schmetz, Bruno, Dipl.-Ing.
(86) Internationale Anmeldenummer: DE9701059
(87) Internationale Veröffentlichungsnummer: WO9745726

(56) Entgegenhaltungen:
- EP-A- 0 740 033
- DE-A- 3 641 875

## Beschreibung

Die vorliegende Erfindung betrifft ein Verfahren zur Messung der Feuchte in Baustoffen sowie weiter eine Vorrichtung zur Durchführung des Verfahrens.

An Baustoffen werden aus verschiedenen Gründen Feuchtemessungen durchgeführt.

Der weitaus größte Anwendungsbereich für die Feuchtemessung an Baustoffen ist die Diagnose von Feuchteschäden. Durch unzureichende Abdichtungen, Havariefälle u.ä. werden in Alt- und Neubauten die Folgen von Feuchteschäden beobachtet. Eine gezielte und somit kostengünstige Instandsetzung erfordert die Kenntnis über die Feuchtequelle, die mit Hilfe von ermittelten Feuchteprofilen gezielt abgestellt werden kann.

Ein anderer Anwendungsbereich ist die Überprüfung der Applikationsmöglichkeit bestimmter Anstriche, Imprägnierungen, Klebern und Mörteln auf Baustoffoberflächen (wie z.B. Oberflächenschutzsysteme in der Betoninstandsetzung, Steinschutzstoffe in der Natursteinfestigung, Injektionsmaterialien für nachträgliche Abdichtungen, wasserabweisende Imprägnierungen, Fliesenkleber). Bei der Applikation ist die Information des Feuchtegehaltes des zu behandelnden Oberflächenbereiches bis zu einer Tiefe von einigen Zentimetern für den Erfolg einer Maßnahme von entscheidender Bedeutung.

Feuchtemessungen werden auch durchgeführt, um die Korrosionsgefahr von Bewehrungsstahl im Betonbau zu beurteilen. Hierbei sind ebenfalls nur die ersten Zentimeter eines Betonbauteiles bzgl. des Feuchtegehaltes interessant. Als Grenzwert kann mit ausreichender Sicherheit eine umgebende Luftfeuchte von rd. 85 % genannt werden, in der sich Korrosionsprozesse nicht mehr in einem bedenklichen Umfang fortsetzen.

Ein weiteres Anwendungsgebiet ist die Überprüfung des örtlichen und zeitlichen Trocknungsverlaufs eines Bauteiles bzw. Bauwerkes (z.B. Wirksamkeitskontrolle einer Instandsetzungsmaßnahme nach Durchführung einer Abdichtungsmaßnahme). Bei fehlgeschlagener Instandsetzung wird so ein rechtzeitiges Eingreifen ermöglicht.

Verfahren und Vorrichtungen zur Feuchtemessung sind prinzipiell bekannt.

Das am weitesten verbreitete Meßverfahren beruht auf der Messung der elektrischen Leitfähigkeit. Gefolgt wird dieses Verfahren von Verfahren, die auf dem sogenannten CM- und dem gravimetrischen Meßprinzip beruhen. Thermometrische Verfahren werden nur vereinzelt an Bauwerken eingesetzt. Kernphysikalische Verfahren (Gammasonde, NMR-Verfahren) werden derzeit nur im Rahmen der Forschung und hier speziell im Laborbereich eingesetzt. Mikrowellenverfahren befinden sich aufgrund ihrer Kalibrierungsproblematik noch im Entwicklungsstadium.

Aus den o.a. Einsatzgebieten ergibt sich, daß ein Verfahren bzw. eine Vorrichtung zur Feuchtemessung baustellentauglich, kostengünstig, schnell, zerstörungsarm, tiefenauflösend und örtlich reproduzierbar sein sollte. Mit Hilfe dieses Anforderungsprofils wurden die in der nachstehenden tabellarischen Übersicht ausgewählten Meßverfahren sowie zugehörige Meßgeräte bewertet.

| Verfahren | | baustellentauglich | kostengünstig | schnell | zerstörungsarm | tiefenauflösend | örtlich reproduzierbar |
|---|---|---|---|---|---|---|---|
| | | Bewertung: positiv ← ++ / + / - /-- → negativ | | | | | |
| Gravimetrisch | Trocknung | ++ | - | - | -- | ++ | -- |
| Chemisch | CM-Methode | ++ | + | + | -- | ++ | -- |
| Thermometrisch | IR-Thermographie | + | - | + | ++ | -- | + |
| Hygrometrisch | Ausgleichsfeuchte | - | - | - | ++ | ++ | ++ |
| Kernphysikalisch | Gamma-Strahlen | - | -- | - | + | ++ | ++ |
| | magn. Kernresonanz | -- | -- | ++ | - | - | - |
| Elektrisch | Leitfähigkeit | - | ++ | ++ | ++ | ++ | - |
| | Mikrowellen | - | - | - | ++ | ++ | ++ |

Aus der Gegenüberstellung derzeit gebräuchlicher sowie in der Forschung befindlicher Feuchtemeßtechniken geht hervor, daß keines der o.a. Meßverfahren allen Anforderungen genügt.

Auf dem Gebiet der hygrometrischen Meßmethode hat lediglich das Fraunhofer Institut für Bauphysik (Holzkirchen) Untersuchungen im Rahmen eines Forschungvorhabens im Jahre 1984 durchgeführt (vgl. DE 36 41 875 A1). Die Meßsensoren verblieben dort dauerhaft im Bohrloch, um die Feuchteverhältnisse in Bauteilen zeitlich lückenlos für Forschungszwecke zu dokumentieren. Aus der genannten Druckschrift geht ebenso wie aus eigenen Erfahrungen mit dem Prototyp hervor, daß eine praktische Anwendung hingegen nur mit sehr großem Aufwand (technisch und wegen des Verbleibs der teueren Meßsensoren im Bohrloch auch finanziell) möglich ist. Insbesondere die Abdichtungslösung mit aufblasbaren Schläuchen mit Hilfe einer Handpumpe ist für den rauhen Baustelleneinsatz nicht geeignet.

Aufgabe der vorliegenden Erfindung ist es daher, unter Vermeidung der aus dem Stand der Technik bekannten Nachteile ein Verfahren und eine Vorrichtung zur Feuchtemessung in Baustoffen zu schaffen, das baustellentauglich (geringe Abmessungen, einfache Handhabbarkeit, einfache Beurteilung der Meßergebnisse vor Ort), kostengünstig (konkurrenzfähig zu handelsüblichen Meßverfahren /-techniken), schnell (Ausgabe des Meßergebnisses unmittelbar nach Messung), zerstörungsarm (kleiner Eingriff in die Bausubstanz), tiefenauflösend (Ermittlung qualitativer Feuchteprofile, Tiefenauflösung > 3 mm) und örtlich reproduzierbar (zur Überprüfung des zeitlichen Trocknungsverlaufes eines Bauteils) ist.

Diese Aufgabe wird mit einem Verfahren gemäß Anspruch 1, einer Meßsonde gemäß Anspruch 2, einem Dichtkörper gemäß Anspruch 4 bzw. einer Vorrichtung gemäß Anspruch 6 gelöst. Vorteilhafte Ausgestaltungen der Erfindung sind Gegenstand der Unteransprüche.

Mit Hilfe der Erfindung können gegenüber den derzeit üblichen Verfahren/Vorrichtungen nachstehende Vorteile erzielt werden:
- baustellentauglich (geringe Abmessungen, einfache Handhabbarkeit, einfache Beurteilung der Meßergebnisse vor Ort); Die Abmessungen liegen aufgrund der ausgereiften Sensortechnik und zugehöriger Auswerteelektronik im Bereich üblicher Handmeßgeräte und sind von einer Person leicht zu bedienen. Kalibriermessungen vor Ort sind nicht erforderlich. Bezüglich der Beurteilung gemessener Daten vor Ort stellt sich bei allen derzeit angewandten Meßverfahren die Frage, was als ,,feuchter Baustoff" einzustufen ist. Die Frage nach einem übermäßig durchfeuchteten Baustoff kann mit den klassischen Feuchtemeßverfahren (gravimetrisch etc.) nicht mit ausreichender Sicherheit und zudem nicht ausreichend schnell beantwortet werden. Für den Innenraumbereich kann man allerdings sogenannte Behaglichkeitsparameter zur Beurteilung heranziehen. Bei Raumtemperaturen von 18 bis 22°C empfindet man eine relative Luftfeuchte von rd. 40% bis zu 75 % als behaglich. Das heißt, daß man mit Hilfe der Messung einer relativen Luftfeuchte am bzw. im Baustoff in der Lage ist, Meßwerte von mehr als 75% als ,,übermäßig" feucht einzustufen. Zudem ist es bei einem in der Regel inhomogenen Bauteil (z.B. Mauerwerk - Ziegel - Mörtel) von großem Vorteil, über die Baustoffgrenzen hinweg eine homogene Feuchteprofilmessung durchzuführen.
- kostengünstig (konkurrenzfähig mit handelsüblichen Meßverfahren /-techniken, kein aufwendiger Eingriff in die Bausubstanz erforderlich);
- schnell (Ausgabe des Meßergebnisses unmittelbar nach der Messung vor Ort); Aus den vorliegenden Forschungsergebnissen geht eindeutig eine Abhängigkeit der Meßdauer (bis zum Einstellen der Gleichgewichtsfeuchte) von der Temperatur und dem Verhältnis von Meßkammervolumen zur entsprechenden Baustoffoberfläche hervor. Bei sehr ungünstigen Temperaturbedingungen (rd. 0 °C Umgebungstemperatur) und einem ungünstigen Volumen/Flächen-Verhältnis wurden Meßzeiten von maximal 45 min. ermittelt, die bei Einsatz von mehreren Meßfühlern durchaus zu tolerieren und wirtschaftlich sind.
- zerstörungsarm (kleiner Eingriff in die Bausubstanz); je nach Ausführung sind Bohrlochdurchmesser von 8 bis 25 mm zur Messung ausreichend;
- tiefenauflösend (Ermittlung qualitativer Feuchteprofile); Die Auflösung kann beliebig bis auf rd. 3 mm reduziert werden;
- örtlich reproduzierbar (zur Überprüfung des zeitlichen Trocknungsverlaufes eines Bauteils); Die Messungen können im gleichen Bohrloch wiederholt werden;

Die Erfindung wird nachstehend anhand eines Ausführungsbeispiels und unter Bezugnahme auf die beigefügten Zeichnungsfiguren näher erläutert, die Folgendes zeigen:
- Fig. 1: zeigt eine im Bohrloch (geschnitten dargestellt) angeordnete erste Ausführungsform einer erfindungsgemäßen Meßsonde zur Messung der Feuchte;
- Fig. 2: zeigt eine im Bohrloch (geschnitten dargestellt) angeordnete zweite Ausführungsform einer erfindungsgemäßen Meßsonde zur Messung der Feuchte;
- Fig. 3: a bis c illustrieren das erfindungsgemäße Verfahren der Feuchtemessung mit der Vorrichtung aus Figur 1 und der Abdichtung des Bohrlochs (geschnitten dargestellt) mit einem Dichtkörper;
- Fig. 4: zeigt eine Teilschnittansicht einer Dichtmanschette gemäß der Erfindung; und
- Fig. 5 a bis d: illustrieren das erfindungsgemäße Verfahren der Feuchtemessung mit einer Meßsonde und der Abdichtung des Bohrlochs (geschnitten dargestellt) mit einer Dichtmanschette gemäß Figur 4;

An dieser Stelle wird zunächst allgemein auf das Meßprinzip eingegangen.

Der Erfindung liegt die Erkenntnis zugrunde, daß durch Messung der relativen Luftfeuchte in einem abgegrenzten Bohrlochabschnitt auf die Feuchtesituation eines Bauteiles geschlossen werden kann. Grundlage ist hier der nahezu temperaturunabhängige Zusammenhang zwischen der relativen Feuchte der den Baustoff umgebenden Luft und dem Baustoff-Feuchtegehalt. Im Labor ermittelte sogenannte Sorptionsisothermen geben Aufschluß über diese baustoffspezifische Korrelation zwischen relativer Luftfeuchte und Baustoff-Feuchte.

Zur Messung der relativen Luftfeuchte werden Feuchtesensoren mit einem kapazitiven Meßprinzip eingesetzt. Diese sind in den letzten Jahren so robust verfügbar geworden, daß nach einer Betauung oder selbst nach einem Eintauchen des Sensors in Wasser keine neue Kalibrierung erforderlich wird.

Zur Messung werden eine oder mehrere Meßkammer(n) im Bohrlochabschnitt zu beiden Seiten jeweils mit einer Dichtung abgegrenzt.

Nach erfolgter bzw. während der Messung (je nach Ausführung) wird das Bohrloch mit einem Dichtkörper derart verschlossen, daß sowohl ein Feuchteaustausch mit der Umgebung außerhalb als auch innerhalb des Bohrloches unterbunden wird und so zu späteren Zeitpunkten wieder Messungen am gleichen Bohrloch durchgeführt werden können. Grundgedanke bei allen in der Folge beschriebenen Ausführungsbeispielen ist also das Wiederverschließen des Bohrloches, so daß Wiederholungsmessungen über einen längeren Zeitraum möglich werden. Die Sensoren werden aus der oben bereits erwähnten technischen und wirtschaftlichen Gründe also nicht auf Dauer im Bohrloch installiert.

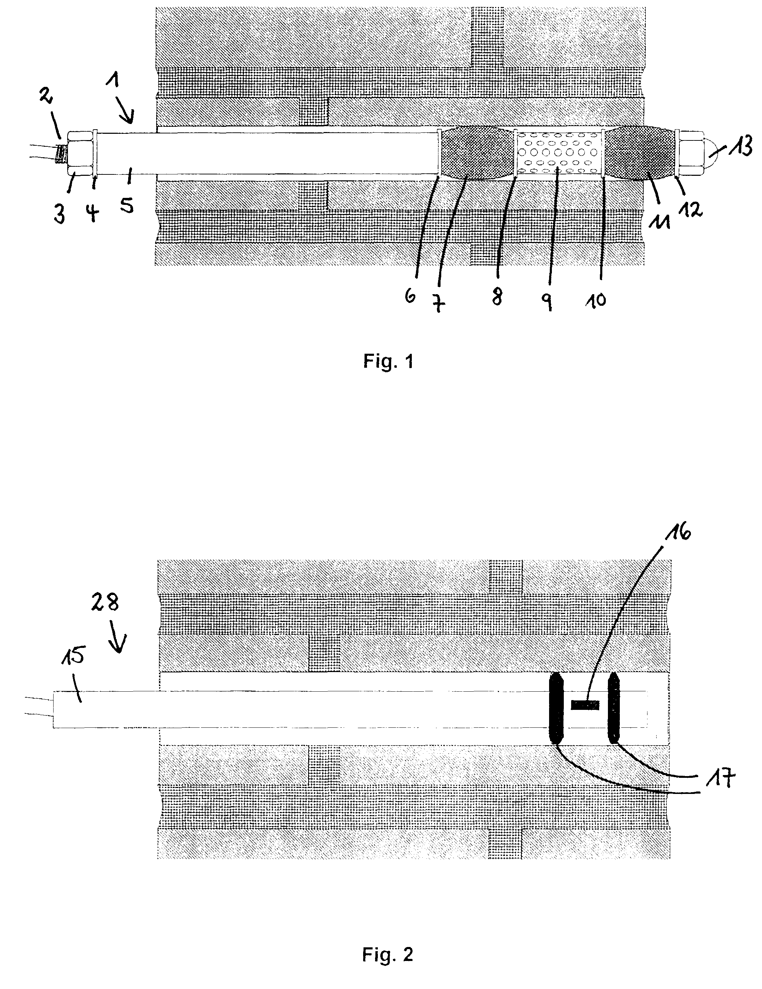

Eine erste Ausführungsform einer Abdichtung ist in Figur 1 dargestellt. Das Mittel zur Abdichtung des Bohrlochs basiert hier im großen und ganzen auf einem Injektionspacker, wie er in der Patent anmeldung EP-A-0 740 033, die nach dem Prioritätsdatum veröffentlicht ist, beschrieben ist. Unterschied zu dem beschrieben Injektionspacker ist lediglich, daß keine Injektionslösung eingeführt wird, sondern ein Sensor bis zu der Stelle zwischen den Abdichtungen eingeführt wird bzw. an dieser Stelle in diesem fest installiert ist.

Dieser Dichtpacker 1 besteht zunächst aus einem Röhrchen 2 mit Außengewinde. Dieses Röhrchen 2 ist an seinem äußeren Ende offen und an seinem inneren Ende durch eine aufgeschraubte Hutmutter 13 verschlossen. Zwischen dem äußeren Ende und der Hutmutter 13 sind nacheinander folgende Bauteile auf das Röhrchen 2 geschraubt bzw. gesteckt: Eine Mutter 3, eine Unterlegscheibe 4, ein Distanzröhrchen 5, eine Unterlegscheibe 6, eine Gummihülse 7, eine Unterlegscheibe 8, eine luftdurchlässige, gelochte Hülse 9, eine Unterlegscheibe 10, eine weitere Gummihülse 11 und eine Unterlegscheibe 12.

Durch Anziehen der Mutter 3 spreizen sich die Gummihülsen 7 und 11 in das Bohrloch hinein. Hervorzuheben ist hier die Verwendung von Gummi- bzw. Kautschukmaterialien, die über ein großes Querdehnungsvermögen verfügen und sich zudem leicht an die rauhe Bohrlochwandung anschmiegen.

Wenn durch das Anziehen des Dichtpackers 1 ein in Bohrlochrichtung eingegrenzter Bereich gebildet ist, kann der Meßvorgang beginnen. Wenn der Sensor nicht fest in dem Dichtpacker 1 installiert ist, muß er soweit in diesen eingeführt werden, daß er sich innerhalb der Hülse 9 befindet und so die Feuchte mißt, die sich durch Austausch des eingegrenzten Bereichs mit dem angrenzenden Baustoff einstellt.

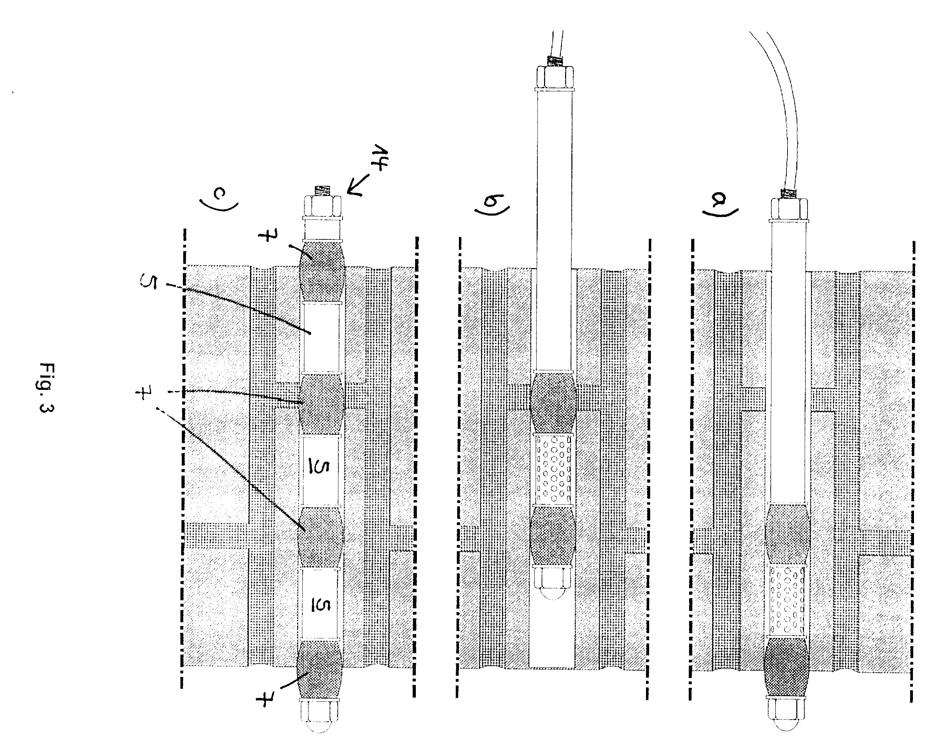

In Figuren 3a und 3b ist dargestellt, wie mit einer derartigen Einrichtung nacheinander der Feuchtegehalt verschiedener Bereiche in verschiedenen Tiefen des Bohrlochs bestimmt werden kann. Die Ermittlung von Meßwerten ist in beliebigen Tiefen des Bohrloches möglich. So sind z.B. bei einem Bohrlochdurchmesser von 25 mm ,,Meßtiefen" von 1 m und mehr möglich.

Weiter ist in Figur 3c gezeigt, wie nach erfolgter Messung das Bohrloch bis zur nächsten Messung wieder verschlossen wird, so daß der weitere Feuchteaustausch der einzelnen untersuchten Bereiche unterbunden wird. Dies erfolgt durch einen Dichtkörper 14, der entsprechend viele Gummihülsen 7 und Distanzröhrchen 5 aufweist und bis zur nächsten Messung im Bohrloch verbleibt.

In Figur 2 ist eine andere Ausführungsform einer Abdichtung 28 gezeigt. Diese besteht im wesentlichen aus einem den Sensor aufnehmenden Röhrchen 15 mit Öffnung 16, die sich zwischen zwei eine Art Meßkammer bildenden Dichtlippen 17 befindet. Der Sensor ermittelt so nur die Feuchte, die sich aufgrund des im eingegrenzten Bereich befindlichen feuchten Baustoffs einstellt. Die Dichtlippen 17 können mit dem Röhrchen 15 verbundene Ringe, kreisförmige Gummischeiben od. dgl. sein.

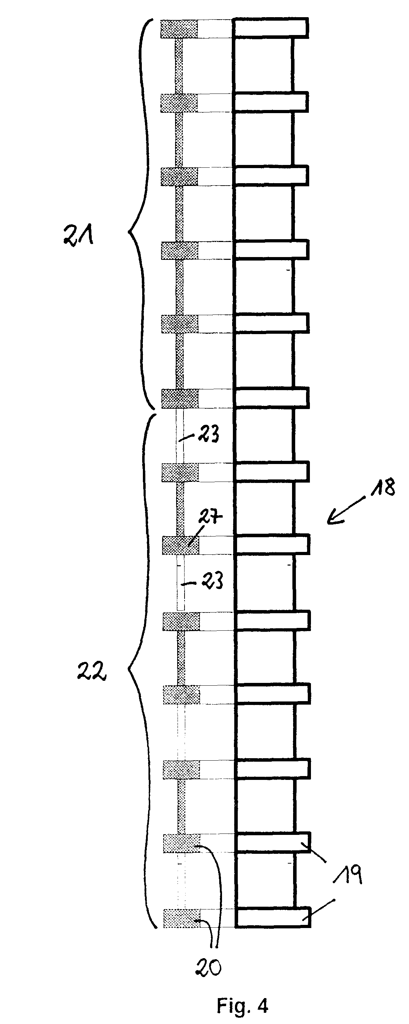

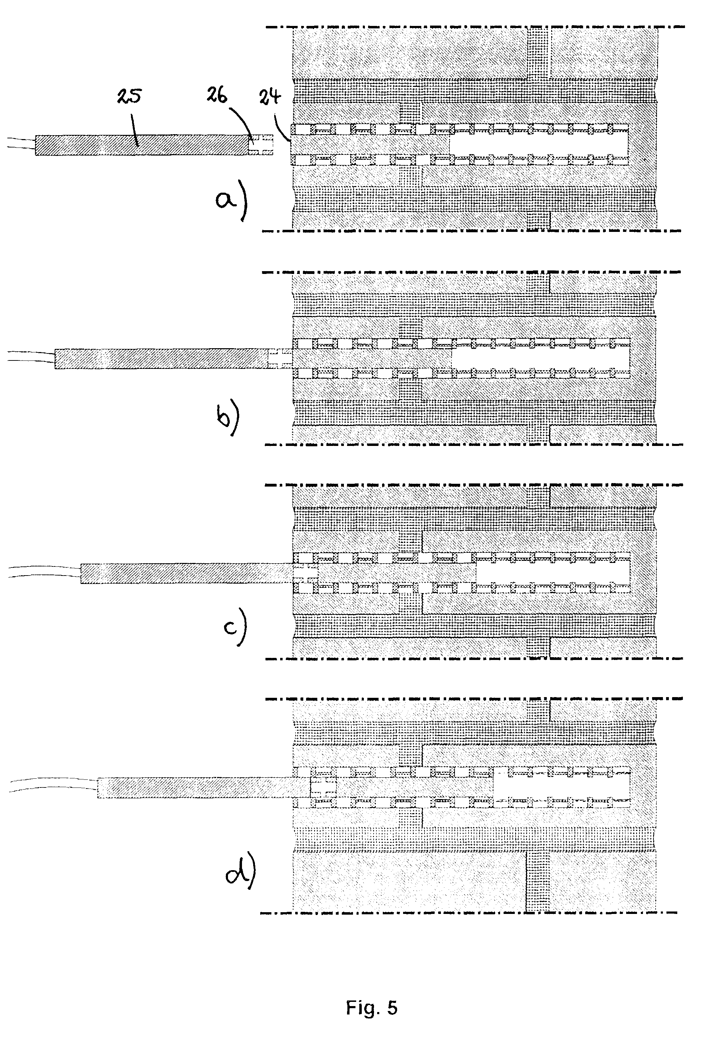

Eine weitere bevorzugte Ausführungsform ist in den Figuren 4 und 5 dargestellt. Diese berücksichtigt die sich im Einzelfall ergebenden Meßdauern von bis zu rd. 45 min, bis sich die Ausgleichsfeuchte im Bohrlochabschnitt eingestellt hat.

Der dargestellte Dichtkörper 18 oder Dichtmanschette besteht aus einem z.B. im Spritzguß hergestellten im großen und ganzen rohrförmigen Gummiteil, das an seiner Außenwandung äußere Dichtlippen oder -lamellen 19 und an seiner Innenwandung innere Dichtlippen oder -lamellen 20 aufweist. Die äußeren Dichtlippen 19 dienen dabei der Abdichtung gegenüber dem Bohrloch und die inneren Dichtlippen 20 dienen der Abdichtung gegenüber dem einzuführenden Sensor. Der Dichtkörper 18 ist im hinteren Bereich 21 geschlossen. Im vorderen Bereich 22 sind hingegen Offnungen 23 vorgesehen, die einen Gasaustausch zwischen der Außenseite des Dichtkörpers 18 und der Innenseite des Dichtkörpers 18 gestatten.

Nachdem der Dichtkörper 18 in das Bohrloch eingeführt worden ist, wird in den Dichtkörper 18, wie in Figur 5a dargestellt, ein Bolzen 24 eingeführt. Hierdurch wird erzielt, daß die Öffnungen 23 gegeneinander abgedichtet werden, so daß sich voneinander isolierte Meßkammern bilden. Der Dichtkörper 18 verbleibt mit dem Bolzen 24 dauerhaft im Bohrloch und wird auch bei der Messung nicht entfernt. Der Meßfühler 25 besitzt keine eigene Abdichtung und nutzt die (inneren) Dichtlamellen 20 des Dichtkörpers 18. Durch Ankopplung des Meßfühlers 25 an den im Inneren des Dichtkörpers 18 befindlichen Bolzens 24 (Figur 5b) und anschließendes Hineinschieben bis zur gewünschten Meßposition (Figur 5c), kann ein Luftaustausch nur zwischen der Sensorkammer 26 und der Meßkammer erfolgen. Alle weiter innen gelegenen Meßkammern bleiben durch die Abdichtung durch den Bolzen 24, alle weiter außen gelegenen Meßkammern bleiben durch die Abdichtung durch den Sensor 25 abgedichtet. Die ,,feuchte Luft" kann in die Sensorkammer 26 einströmen, wobei sich die Meßdauer in Abhängigkeit von dem im Dichtkörper 18 vorhandenen Anteil an feuchter Luft im Verhältnis zum Sensorkammervolumen ergibt. Hieraus resultieren weitaus geringere als die oben erwähnten Meßzeiten.

Nach Abschluß der Messung wird der Sensor 25 mit dem angekuppelten Bolzen 24 wieder aus dem Bohrloch herausgezogen und von diesem getrennt. Der Bolzen verbleibt dabei im vorderen Teil des Bohrloches und dichtet die Meßkammern bis zur nächsten Messung, die unter Umständen nach Monaten erfolgen kann, ab.

Da auch die Eintrittsöffnung der Sensorkammer 26 eine gewisse Ausdehnung aufweist, ist darauf zu achten, daß die zwischen den einzelnen Offnungen 23 befindlichen Trennwände oder Stege 27 nicht zu klein sind. Andernfalls kommt es im Moment des Einschiebens oder Herausziehens zu einem Gasaustausch zwischen benachbarten Kammern. Weiterhin muß dann die Positionierung sehr genau erfolgen, um einen Anschluß an die gewünschte Meßkammer herzustellen. Aus diesem Grunde befinden sich bei der in Figur 4 dargestellten Ausführungsform verhältnismäßig große Abstände zwischen den einzelnen Offnungen 23. Beim Weiterschieben des Sensors von einer Meßkammer 23 zur benachbarten Meßkammer 23 kann es nicht zu einem Gasaustausch kommen, da der Sensor vor Eintritt in die nächste Meßkammer 23 die vorherige bereits vollständig verlassen hat, wie in Figur 5d dargestellt ist.

Das vorstehend erläuterte Verfahren und die anhand bevorzugter Ausführungsformen beispielhaft erläuterte erfindungsgemäße Vorrichtung zur Durchführung des Verfahrens löst die oben gestellte Aufgabe in einfacher und besonders wirtschaftlicher Weise.

### BEZUGSZIFFERNLISTE

- 1: Dichtpacker
- 2: Röhrchen
- 3: Mutter
- 4: Unterlegscheibe
- 5: Distanzröhrchen
- 6: Unterlegscheibe
- 7: Gummihülse
- 8: Unterlegscheibe
- 9: gelochte Hülse
- 10: Unterlegscheibe
- 11: Gummihülse
- 12: Unterlegscheibe
- 13: Hutmutter
- 14: Dichtkörper
- 15: Röhrchen
- 16: Offnung
- 17: Dichtlippen
- 18: Dichtkörper
- 19: Dichtlippen oder -lamellen
- 20: Dichtlippen
- 21: hinteren Bereich
- 22: vorderen Bereich
- 23: Offnungen
- 24: Bolzen
- 25: Meßfühler
- 26: Sensorkammer
- 27: Trennwände (Stege)
- 28: Abdichtung

## Patentansprüche

1. Verfahren zur Messung der Feuchte in Baustoffen, umfassend die folgenden Verfahrensschritte:
1.1. Einbringen eines Bohrloches in den Baustoff,
1.2. Bildung von in Bohrlochrichtung eingegrenzten, den Gasaustausch mit den angrenzenden Meßkammern weitgehend unterbindenden Meßkammern in dem Bohrloch,
1.3. Messung der Feuchte in jeder Meßkammer mittels Meßsonden;
**dadurch gekennzeichnet,**
1.4. daß zur Messung der Feuchte eine Meßsonde zeitversetzt mit jeder der Meßkammern verbunden wird;
1.5. daß die Meßsonde aus dem Bohrloch entfernt; und
1.6. daß die Meßkammern für zukünftige Messungen mittels eines Dichtkörpers so verschlossen werden, daß der Gasaustausch mit den angrenzenden Meßkammern weitgehend unterbunden wird.

2. Meßsonde zum Einsatz bei dem Verfahren nach Anspruch 1, dadurch gekennzeichnet, daß sie Mittel (7, 11; 17) zur Abdichtung des Bohrlochs und zur Bildung einer Meßkammer in dem Bohrloch aufweist.

3. Meßsonde nach Anspruch 2, dadurch gekennzeichnet, daß die Mittel (7, 11; 17) zur Abdichtung des Bohrlochs und zur Bildung einer Meßkammer in dem Bohrloch bestehen aus:
- einem Röhrchen (2) mit Außengewinde;
- einer auf das Röhrchen aufgeschraubten Muttern (3) und einer auf das Röhrchen aufgeschraubten Hutmutter (13);
- zwischen der Mutter (3) und der Hutmutter (13) auf dem Röhrchen (2) mittels gelochter Hülse (9) beabstandet angeordneten Gummihülsen (7, 11), deren Durchmesser durch Drehung der Mutter (3) veränderbar sind.

4. Dichtkörper zum Einsatz bei dem Verfahren nach Anspruch 1, dadurch gekennzeichnet, daß er in das Bohrloch einsetzbar ist und Mittel (7) zur Abdichtung des Bohrlochs und Bildung von Meßkammern in dem Bohrloch aufweist und zur Durchführung der Messung mit einer Meßsonde gemäß Anspruch 2 oder 3 aus dem Bohrloch entfernbar ist.

5. Dichtkörper nach Anspruch 4, dadurch gekennzeichnet, daß die Mittel (7, 11; 17) zur Abdichtung des Bohrlochs und zur Bildung von Meßkammern in dem Bohrloch bestehen aus:
- einem Röhrchen (2) mit Außengewinde;
- einer auf das Röhrchen aufgeschraubten Muttern (3) und einer auf das Röhrchen aufgeschraubten Hutmutter (13);
- zwischen der Mutter (3) und der Hutmutter (13) auf dem Röhrchen (2) mittels gelochter Hülse (9) beabstandet angeordneten Gummihülsen (7, 11), deren Durchmesser durch Drehung der Mutter (3) veränderbar sind.

6. Vorrichtung zum Einsatz bei dem Verfahren nach Anspruch 1, dadurch gekennzeichnet,
- daß sie eine in das Bohrloch einsetzbare, flexible, sich mit äußeren Dichtlippen (19) an die Bohrlochwandung und mit inneren Dichtlippen (20) an einen in sie eingesetzten Bolzen (24) anschmiegende Dichtmanschette (18) aufweist, die in ihrem vorderen Bereich (22) Öffnungen (23) aufweist, die einen Gasaustausch zwischen der Außenseite der Dichtmanschette (18) und der Innenseite der Dichtmanschette (18) gestattet, wodurch Meßkammern gebildet werden, und
- daß der Bolzen (24) eine Kupplung zum Anschließen einer Meßsonde (25) aufweist und zusammen mit dieser in der Dichtmanschette (18) verschiebbar ist.

## Claims

1. Method for measuring the moisture in building materials comprising the following procedure steps:
1.1. provision of a borehole in the building material,
1.2. formation of measuring chambers in the borehole which are delimited in the direction of the borehole and substantially prevent the exchange of gas with the adjacent measuring chambers,
1.3. measurement of the moisture in each measuring chamber by means of measuring probes:
characterised in that
1.4. a measuring probe is connected at different times to each of the measuring chambers in order to measure the moisture,
1.5. in that the measuring probe is removed from the borehole; and
1.6 in that, for future measurements, the measuring chambers are closed by means of a sealing body such that the exchange of gas with the adjacent measuring chambers is substantially prevented.

2. Measuring probe for use with the method according to claim 1, characterised in that said probe has means (7, 11; 17) for sealing the borehole and for forming a measuring chamber in the borehole.

3. Measuring probe according to claim 2, characterised in that the means (7, 11; 17) for sealing the borehole and for forming a measuring chamber in the borehole comprise:
• a small tube (2) with an external thread;
• a nut (3) which is screwed onto the small tube and a cap nut (13) which is screwed onto the small tube;
• rubber bushes (7, 11) which are disposed at a spacing between the nut (3) and the cap nut (13) on the small tube (2) by means of the perforated sleeve (9) and the diameters of which bushes (7, 11) can be changed by rotating the nut (3).

4. Sealing body for use with the method according to claim 1, characterised in that said sealing body can be inserted into the borehole and has means (7) for sealing the borehole and for forming measuring chambers in the borehole and can be removed from the borehole for performing the measurement with a measuring probe according to claim 2 or 3.

5. Sealing body according to claim 4, characterised in that the means (7, 11; 17) for sealing the borehole and for forming measuring chambers in the borehole comprise:
• a small tube (2) with an external thread;
• a nut (3) which is screwed onto the small tube and a cap nut (13) which is screwed onto the small tube;
• rubber bushes (7, 11) which are disposed at a spacing between the nut (3) and the cap nut (13) on the small tube (2) by means of the perforated sleeve (9) and the diameters of which bushes (7, 11) can be changed by rotating the nut (3).

6. Device for use with the method according to claim 1, characterised in that
• said device has a sealing sleeve (18) which can be inserted into the borehole, is flexible and adapts with exterior sealing lips (19) to the borehole wall and, with interior sealing lips (20), to the bolt (24) inserted therein, which sealing sleeve has openings (23) in its front region (22) which permit an exchange of gas between the outside of the sealing sleeve(18) and the inside of the sealing sleeve (18), by means of which measuring chambers are formed, and
• in that the bolt (24) has a coupling for connecting a measuring probe (25) and is slideable in the sealing sleeve (18) together with said probe.

## Revendications

1. Procédé de mesure de l'humidité dans des matériaux de construction, comprenant les étapes de procédé suivantes :
1.1. Perçage d'un trou de sondage dans le matériau de construction,
1.2. Formation dans le trou de sondage de chambres de mesure délimitées dans la direction du trou de sondage et empêchant pratiquement l'échange gazeux avec les chambres de mesure adjacentes,
1.3. Mesure de l'humidité dans chaque chambre de mesure au moyen de sondes de mesure ;
**caractérisé**
1.4. en ce que, pour mesurer l'humidité, une sonde de mesure est reliée successivement à chaque chambre de mesure ;
1.5. en ce que la sonde de mesure est sortie du trou de sondage ; et
1.6. en ce que les chambres de mesure sont bouchées par un corps d'étanchéité pour des mesures postérieures de telle sorte que l'échange gazeux avec les chambres de mesure adjacentes soit pratiquement empêché.

2. Sonde de mesure destinée à être utilisée dans le procédé selon la revendication 1, **caractérisés en ce qu**'elle présente des moyens (7, 11, 17) pour isoler le trou de sondage et pour former une chambre de mesure dans le trou de sondage.

3. Sonde de mesure selon la revendication 2, **caractérisé en ce que** les moyens (7, 11, 17) pour isoler le trou de sondage et pour former une chambre de mesure dans le trou de sondage sont constitués :
- d'un tube (2) avec filetage extérieur ;
- d'un écrou (3) vissé sur le tube et d'un écrou borgne (13) vissé sur le tube ;
- de manchons en caoutchouc (7, 11) placés sur le tube (2) entre l'écrou (3) et l'écrou borgne (13), séparés au moyen de manchons percés (9) et dont le diamètre peut être modifier en tournant l'écrou (3).

4. Corps d'étanchéité destiné à être utilisé dans le procédé selon la revendication 1, **caractérisé en ce qu**'il peut être introduit dans le trou de sondage et qu'il présente des moyens (7) pour isoler le trou de sondage et pour former des chambres de mesure à l'intérieur du trou de sondage et qu'il peut être enlevé du trou de sondage dans le but de réaliser la mesure avec une sonde de mesure selon la revendication 2 ou 3.

5. Corps d'étanchéité selon la revendication 4, **caractérisé en ce que** les moyens (7, 11, 17) pour rendre étanche le trou de sondage et pour former des chambres de mesure dans le trou de sondage sont constitués :
- d'un tube (2) avec filetage extérieur ;
- d'un écrou (3) vissé sur le tube et d'un écrou borgne (13) vissé sur le tube ;
- de manchons en caoutchouc (7, 11) placés sur le tube (2) entre l'écrou (3) et l'écrou borgne (13), séparés au moyen de manchons percés (9) et dont le diamètre peut être modifié en tournant l'écrou (3).

6. Dispositif destiné à être utilisé dans le procédé selon la revendication 1, **caractérisé**
- en ce qu'il est muni d'une manchette d'étanchéité (18) flexible pouvant être introduite dans le trou de sondage et se plaquant avec des lèvres d'étanchéité externes (19) contre la paroi du trou de sondage et avec des lèvres d'étanchéité internes (20) contre un boulon (24) introduit à l'intérieur, ladite manchette étant munie dans sa section avant (22) d'ouvertures (23) permettant un échange gazeux entre la face externe de la manchette d'étanchéité (18) et la face interne de la manchette d'étanchéité (18), de sorte que des chambres de mesure soient formées, et
- en ce que le boulon (24) présente un raccord pour brancher une sonde de mesure (25) et qu'ils sont déplaçables ensemble à l'intérieur de la manchette d'étanchéité (18).
